# EUROPEAN PATENT SPECIFICATION

(11) **EP 2 692 340 B1**
(45) Date of publication and mention of the grant of the patent: **11.03.2015**
(21) Application number: 13386023.9
(22) Date of filing: 29.07.2013
(51) Int. Cl.: A61K 9/14, A61K 47/32, A61K 31/198

(54) **Stable pharmaceutical system (kit) for the preparation of oral solution of levothyroxine or pharmaceutically acceptable salt thereof**
Stabiles pharmazeutisches System (Kit) zur Herstellung einer oralen Lösung aus Levothyroxin oder einem pharmazeutisch unbedenklichen Salz davon
Système pharmaceutique stable (kit) pour la préparation de solution administrée par voie orale de lévothyroxine ou d'un sel pharmaceutiquement acceptable de celle-ci

(30) Priority: 03.08.2012 GR 20120100410
(43) Date of publication of application: 05.02.2014
(73) Proprietor: Verisfield (UK) Ltd., 152 31 Halandri Attikis (GR)
(72) Inventor: Koutsodimos, Christos, 152 31 Halandri Attikis (GR); Motsios, Georgios, 152 31 Halandri Attikis (GR)

(56) References cited:
- WO-A1-03/041700
- GR-A- 20100 100 508
- JARROD W. COLLIER ET AL: "Influence of Formulation and Processing Factors on Stability of Levothyroxine Sodium Pentahydrate", AAPS PHARMSCITECH, vol. 11, no. 2, 8 May 2010 (2010-05-08), pages 818-825, XP55043533, DOI: 10.1208/s12249-010-9434-8

## Description

### FIELD OF THE INVENTION

The present invention relates to a stable pharmaceutical system (kit) for the preparation of oral solution of levothyroxine or pharmaceutically acceptable salt thereof, which consists of a solid for reconstitution in the form of powder or granules and of a reconstitution liquid.

### BACKGROUND OF THE INVENTION

Levothyroxine and its salts are active substances with proven therapeutic value and are used by numerous patients worldwide (this point onwards the term levothyroxine will also include the salts of the active ingredient). It is well known that thyroid hormones are used in cases of absent or diminished thyroid action, as a replacement therapy. In the vast majority of cases, solid dosage forms are administered, despite the fact that they are unable to provide a flexible dosing system while the dosage applied in medical practice ranges from 25mcg to more than 125mcg per dose. It is obvious that a flexible system of variable doses of levothyroxine would be preferable. The reason behind the selection of solid dosage forms is the problematic stability of levothyroxine in liquid media, which does not allow the development and production of liquid formulations with adequate shelf life. These products suffer from two major defects, namely the increased degradation of the active ingredient and its precipitation from oral solutions. The latter cannot be observed by visual inspection due to the low concentration of the active ingredient and it is thus very difficult for the end user to identify a non conforming product, wherein precipitation of the active ingredient has taken place. Moreover, some of the proposed liquid formulations require storage at low temperatures, which makes the use of the product inconvenient to the patients, while increasing storage and distribution costs.

The actual content of levothyroxine and possible degradation products in formulations thereof, has been in the focus of regulatory discussions during the last years; the US Food and Drug Administration (FDA) has recently issued a directive limiting the allowed range of the amount of levothyroxine in its formulations from 90-110% to 95-105% of the nominal amount per product. This decision was taken in order to improve the quality of levothyroxine formulations and lead the manufacturers towards actions that will reduce the variability of their formulations and processes. This will eventually improve the efficacy of such products and mitigate any concerns of physicians and patients (http://www.fda.gov/Drugs/DrugSafety/PostmarketDrugSafetyInformationforPatientsa ndProviders/ucm 161266. htm).

An additional technical issue in manufacturing levothyroxine formulations is to achieve uniformity in the content of the active ingredient in the final products, considering that only a few micrograms of active ingredient should be evenly dispersed in granules or powders which will be compressed into tablets or filled into capsules.

To date, very few attempts have been conducted to develop liquid levothyroxine formulations, so the bibliographic data available and the prior art mainly relate to solid dosage forms of said active ingredient. It should be noted that in several cases the conclusions in the literature concerning the stability of levothyroxine are confusing and even contradictory.

For example, EP 0 742 714 B1 discloses a stable liquid formulation of levothyroxine, comprising 40%-96% ethanol by volume, a buffer regulating in the area 9-12 and water 4%-50% by volume. Formulations comprising up to 5% by mass of a sequestrating agent and up to 5% by mass of an antioxidant are also claimed. It should be stressed, however, that the administration of organic solvents is not desirable in therapeutics, especially for long-term or lifetime treatments, as is the case with thyroid hormone replacement therapy. It should also be noted that the effect of pH value on levothyroxine stability described is in agreement with some reports in the literature presented later on in this application (Glass and Heywood, 2006, Won, 1992, Patel et al, 2003, Das Gupta et al, 1990), but in contrast with others (Clarke's Analysis of Drugs and Poisons, 2005). The use of Polyvinyl pyrrolidone (PVP) is another point of conflict, supported by EP 0 742 714 B1 but rejected by Collier et al, 2010.

US 5225204 discloses a stable dosage form of levothyroxine and a manufacturing process thereof, said form comprising a complex of levothyroxine with a water soluble polyvinyl pyrrolidone adsorbed on a cellulose compound. The dosage form is a tablet or a gelatin capsule filled with granules and the cellulose compound in the final formulation in tablets or in gelatin capsules is selected from the group of microcrystalline cellulose, hydroxypropyl cellulose, hydroxyethyl cellulose, sodium carboxymethyl cellulose and hydroxypropylmethyl cellulose. The same document also claims levothyroxine complexes with block copolymers of ethylene oxide and propylene oxide adsorbed on a cellulose compound in a similar way.

US 5635209 discloses a stabilized formulation of levothyroxine and the manufacturing process thereof, which includes a combination of levothyroxine with a carrier (microcrystalline cellulose), potassium iodide mixed with the same carrier (microcrystalline cellulose), a disintegrant and a lubricant. The data in the literature concerning the stability of levothyroxine are contradictory also in this case, as microcrystalline cellulose is considered an inappropriate excipient by Collier et al, 2010 and Patel et al, 2003.

Collier et al, (Collier et al, 2010, "Influence of Formulation and Processing Factors on Stability of Levothyroxine Sodium Pentahydrate", AAPS PharmSciTech) have recently studied the stability of levothyroxine at relatively high temperatures (>60°C) which accelerate the degradation of said active ingredient, in the presence of various excipients. They found that levothyroxine in solid state was stable both during normal and accelerated thermal stability tests (0% relative humidity at a temperature of 25°C or 40°C), but degraded significantly (almost 40%) in the presence of humidity when exposed to higher temperatures. These findings are obviously not favorable for the development of liquid formulations of levothyroxine with an adequate shelf life. The same document also discloses certain excipients whose presence in high humidity conditions leads to the degradation of levothyroxine at various degrees. For example, a practically complete degradation of levothyroxine is reported in the presence of crospovidone, povidone and sodium lauryl sulfate. Degradation at an intermediate degree (around 50%) was caused by the interaction of levothyroxine with Mannitol, Microcrystalline Cellulose, and Sucrose. The smallest amount of degradation, the active ingredient remaining at the end of a 28-day trial exceeding 50%, was observed with Colloidal Silica Dioxide, Magnesium stearate, Acacia, Lactose Monohydrate, Croscarmellose Sodium, and Sodium Starch Glycollate. It is obvious that some of these results are not in line with findings described in other documents already mentioned. However, it is an undeniable common conclusion that levothyroxine is unstable in aqueous environment or even in high relative humidity conditions, which renders the preparation of a stable liquid formulation of levothyroxine an important technical challenge.

Glass and Haywood, (Glass and Haywood, 2006, "Stability considerations in liquid dosage forms extemporaneously prepared from commercially available products." J Pharm Pharmaceut Sci, 9, 3:398-426) have also studied the stability of levothyroxine in liquid formulations. According to this document, thyroxine generally appears to be unstable when exposed to light, heat and humidity. This has led to recommendations for the storage of tablets with the specific active ingredient at a temperature of 4-8 °C and also to proposing specific procedures for the administration to paediatric patients and generally in cases of patients with dysphagia, where it is recommended that tablets are crushed and dispersed in 10ml - 20ml of water or other suitable carrier (e.g. breast milk) and the preparation is used immediately. The latter recommendation comes from the fact that when crushing and dispersing the tablets in water, the resulting preparation is unstable, despite the presence of a preservative (methyl paraben) or storage at a temperature of 4-8 °C. Said dispersions contained 40% glycerol and proved that the use of preservatives may have a negative impact on the chemical stability of the active ingredient. This fact is extremely interesting, since it renders the preservation of liquid preparations of levothyroxine through the use of those excipients over an extended period of time difficult.

Patel et al and Das Gupta et al (Patel et al, 2003, "The effect of excipients on the stability of levothyroxine sodium pentahydrate tablets", International Journal of Pharmaceutics 264, 35-43, Das Gupta et al, 1990, "Effect of Excipients on the Stability of Levothyroxine Sodium Tablets", Journal of Clinical Pharmacy and Therapeutics, 15,33 1-336) have suggested the use of basic pH regulators as a potential method of improving the stability of levothyroxine sodium pentahydrate tablets, in combination with the use of excipients with low water content such as dibasic calcium phosphate. This research attempt has also reached the conclusion that humidity control is a key factor for the stability of the active ingredient.

From the prior art mentioned above it is clear that the development of a stable levothyroxine product, which at the time of administration would be in a liquid form and which would allow flexibility in the determination of the dosage e.g. by using a dosing pipette, was an important technical challenge, faced by the present invention.

### SUMMARY OF THE INVENTION

The present invention relates to a stable pharmaceutical system (kit) for the preparation of oral solution of levothyroxine or pharmaceutically acceptable salt thereof, which consists of a solid for reconstitution in the form of powder or granules that comprises levothyroxine or pharmaceutically acceptable salt thereof and of a reconstitution liquid for preparing the oral solution.

The solid for reconstitution, which contains levothyroxine, further comprises at least one excipient-carrier of the active ingredient, preferably water soluble, even more preferably sorbitol or maltodextrin or a mixture thereof.

Said solid for reconstitution is manufactured via a wet granulation process in order to facilitate the dispersion of the active ingredient within the mass of the solid carrier excipients, preferably utilizing the fluidized bed approach.

The reconstitution liquid is aqueous and further comprises at least one excipient that allows the solubilisation of the active ingredient. Preferably polyvinyl pyrrolidone is used as a solubilizer of the active ingredient, providing an aqueous solution that can be used for the complete reconstitution of the solid which includes the active ingredient, according to the present invention.

The pharmaceutical system (kit) of the present invention has the following advantages:
i. The active ingredient, which is susceptible to degradation in high relative humidity conditions or in liquid dosage forms, is in a solid state during long-term storage and is reconstituted prior to use by the patients. The reconstituted liquid product is stable for an adequate period of time, allowing the use of its entire quantity by the patients.
ii. The reconstituted solution has all the advantages of liquid dosage forms. More specifically, the ease of administration to special populations (such as paediatric and geriatric patients) and in cases of patients with dysphagia, as well as the flexibility with regard to the preparation of different doses from the same starting solution, e.g. using a graduated pipette.
iii. Keeping the system (kit) of the present invention in liquid form for a short period of time, only during the use of the product, eliminates the need for adopting special storage and distribution conditions.
iv. The reconstituted product resulting from the system (kit) of the present invention is free of organic solvents and co-solvents which have been proposed in the prior art. This is an advantage of the present invention when taking into consideration the fact that the therapeutic use of levothyroxine is usually long-term and in many cases for life.
v. The manufacturing process of the solid for reconstitution according to the present invention also offers significant advantages in terms of the uniformity of content of the active ingredient in the final formulation. The process of wet granulation by spraying in fluidized bed is the optimum choice, when the uniform dispersion of small quantities of active ingredient in the mass of the final product is sought. However, even the adoption of this technique may have poor results in achieving a uniform dispersion of levothyroxine, when the final dosage unit is of low mass, as is the case with tablets which typically weigh between 100mg and 300mg.

On the contrary, in the present invention, significant quantities of solid are filled in vials, mitigating the risk of poor content uniformity of the active ingredient in the final formulation. For example, for 150ml of reconstituted solution, corresponding to 30 doses of 125mcg, 10-15g of solids are filled in a vial and are reconstituted with an appropriate volume of liquid. It is obvious that, compared to other dosage forms such as tablets, it is easier to achieve content uniformity at this scale of solids' usage.

In conclusion, it is clear that the system (kit) of the present invention is an attractive approach for the production of flexible and stable dosage forms of levothyroxine, which at the time of administration are in liquid form and exhibit an improved stability with respect to other liquid dosage forms of the same active ingredient.

### DETAILED DESCRIPTION OF THE INVENTION

The present invention relates to a stable pharmaceutical system (kit) for the preparation of oral solution of levothyroxine or pharmaceutically acceptable salt thereof, which consists of a solid for reconstitution in the form of powder or granules that comprises levothyroxine or pharmaceutically acceptable salt thereof and of a reconstitution liquid for preparing the oral solution.

The solid for reconstitution, which contains levothyroxine, further comprises at least one excipient-carrier of the active ingredient, preferably water soluble, even more preferably sorbitol or maltodextrin or a mixture thereof, and optionally a binder, preferably polyvinyl pyrrolidone.

Said solid for reconstitution is manufactured via a wet granulation process in order to facilitate the dispersion of the active ingredient within the mass of the solid carrier excipients, preferably utilizing the fluidized bed approach.

The reconstitution liquid is aqueous and further comprises at least one excipient that allows the solubilisation of the active ingredient. Preferably polyvinyl pyrrolidone is used as a solubilizer of the active ingredient, providing an aqueous solution suitable for the complete and effective reconstitution of the solid system (kit) of the present invention.

In more detail the solid for reconstitution according to the present invention comprises:
i. Levothyroxine or a salt thereof, at a quantity ranging from 0.01 to 1.00% w/w, preferably from 0.02 to 0.50% w/w and even more preferably from 0.02 to 0.10% w/w in the solid for reconstitution.
ii. At least one solid excipient-carrier ensuring the stability of the active ingredient, preferably water soluble and even more preferably sorbitol or maltodextrin or a mixture thereof. The amount of the solid excipient-carrier ranges from 30.00 to 99.99% w/w and more preferably from 97.90 to 99.48% w/w in the solid for reconstitution. Excipients of this category allow the preparation of clear solutions after reconstitution, which are stable during the period of use of the final product.

The solid for reconstitution to an oral solution according to the present invention may optionally further comprise:
i. A binder that is used for a more effective dispersion of the active ingredient within the mass of the excipient-carrier during the manufacturing process of the solid for reconstitution, preferably a water soluble binder, which produces water soluble powders after the granulation step, more preferably polyvinyl pyrrolidone. The amount of the binder ranges from 0.01 to 5.00% w/w and more preferably from 0.50 to 2.00% w/w in the solid for reconstitution.
ii. Other common excipients used in this type of products, known from the state of the art, such as sweeteners, taste enhancers, water soluble lubricants, water soluble diluents, viscosity regulators and pH adjusting systems. However, due to the fact that the end product also comprises a reconstitution liquid, some of the excipients mentioned above may be added to the latter, resulting in that the solid for reconstitution has a most simple composition, thus minimizing any potential risk for the stability of the active ingredient therein.

The reconstitution liquid of the system (kit) of the present invention is aqueous and further comprises a solubilizing agent, preferably polyvinyl pyrrolidone, at a quantity ranging from 0.01 to 15.00% w/w, preferably from 2.50 to 7.50% w/w in the reconstitution liquid.

The reconstitution liquid of the system (kit) of the present invention may optionally further contain other common excipients known from the state of the art, such as preservatives, sweeteners, taste enhancers, water soluble lubricants, water soluble diluents, viscosity regulators and pH adjusting systems, as long as they do not have a negative influence on the stability of the final formulation until completion of its use. The system (kit) of the present invention has the following advantages:
i. The active ingredient, which is susceptible to degradation in high relative humidity conditions or in liquid dosage forms, is in a solid state during long-term storage and is reconstituted prior to use by the patients. The reconstituted liquid product is stable for an adequate period of time covering the completion of its use by the patients. All the constituents that might affect the long-term stability of the active ingredient, such as preservatives, sweeteners and solubilising agents are included in the reconstitution liquid and thus come in contact with the active ingredient only during the short period of time that the product is being used.
ii. The reconstituted solution has all the advantages of liquid dosage forms. More specifically, it ensures the ease of administration to special populations (such as paediatric and geriatric patients), and also the flexibility with regard to the preparation of different doses from the same starting solution, using for example a reconstituted solution of 25mcg/ml and taking different doses with a graduated pipette.
iii. Keeping the system (kit) of the present invention in liquid form for a short period of time, only during the use of the product, eliminates the need for adopting special storage and distribution conditions. For example, suggested approaches require at least storage in a refrigerator, which results in patient inconvenience and increased storage and distribution costs.
iv. The reconstituted product resulting from the system (kit) of the present invention is free of organic solvents and co-solvents which have been proposed in the prior art. This is an advantage of the present invention when taking into consideration the fact that the therapeutic use of levothyroxine is usually long-term and in many cases for life. Solvents such as ethanol and propylene glycol may be irritating after long-term use, and may also affect the mobility of the gastrointestinal tract. The system (kit) of the present invention is free of all these deficiencies, since an aqueous solution is produced after reconstitution.
v. The manufacturing process of the system (kit) of the present invention also offers significant advantages in terms of the uniformity of content of the active ingredient in the final formulation. Due to its intrinsic characteristics, the process of wet granulation by spraying in fluidized bed is the optimum choice, when the uniform dispersion of small quantities of active ingredient in the mass of the final product is sought. However, even the adoption of this technique may have poor results in achieving a uniform dispersion of levothyroxine, when the final dosage unit is of low mass, as is the case with tablets which typically weigh between 100mg and 300mg. On the contrary, in the present invention, significant quantities of solid are filled in vials, mitigating the risk of poor content uniformity of the active ingredient in the final formulation. For example, for 150ml of reconstituted solution, corresponding to 30 doses of 125mcg, 10-15g of solids are filled in a vial and are reconstituted with an appropriate volume of liquid. It is obvious that, compared to other dosage forms such as tablets, it is easier to achieve content uniformity at this scale of solids' usage.

The fluidized bed technique is known from the state of the art, as it is described in many technical handbooks. The solid for reconstitution according to the system (kit) of present invention can be produced using any of the fluidized bed alternative methods, i.e. top spray, bottom spray or their combinations, with similar results.

In a preferred embodiment of the present invention the manufacturing process of the solid for reconstitution includes the following steps and parameters:
The product chamber is filled with an appropriate amount of the solid carrier, preferably sorbitol or maltodextrin. The granulation liquid is prepared by dispersing the binder and the active ingredient in water under stirring or mild homogenization. The solid carrier is set in motion by an air flow and/or mechanical action, depending on the type of the fluidized bed apparatus selected. The granulation liquid is sprayed on the solid carrier at a rate typically ranging from 0.01 to 0.10g/min/g_{solid carrier}, in order to ensure optimal conditions during granulation, in a manner known from the state of the art. The incoming air temperature is typically set at 40-60°C, resulting in a product temperature of 32-38°C during spraying of the binding material. Similar product temperatures are utilized during drying, which is performed very fast due to the low water adsorption by the selected carriers, compared for example with the cases of cellulosic or starch based materials. This is a favourable part of the process as levothyroxine is thermosensitive and its processing time is minimized.

An illustrative embodiment of the system (kit) of the present invention comprises a solid for reconstitution into an oral solution and a reconstitution liquid as follows:
a) A solid for reconstitution in the form of a powder for preparing an oral solution comprising:
   i. Levothyroxine or a salt thereof, at a quantity ranging from 0.02 to 0.1 % w/w in the solid for reconstitution.
   ii. At least one solid excipient-carrier ensuring the stability of the active ingredient, such as sorbitol or maltodextrin or a mixture thereof, at a quantity ranging from 90.00-98.00% w/w in the solid for reconstitution.
   iii. A binder used to disperse the active ingredient within the mass of the solid carrier during the manufacturing process, such as polyvinyl pyrrolidone, at a quantity ranging from 0.50 to 2.00% w/w in the solid for reconstitution.
b) An aqueous reconstitution liquid comprising:
   i. A solubilizing agent of the active ingredient, such as polyvinyl pyrrolidone, at a quantity ranging from 2.50 to 7.50% w/w in the reconstitution liquid.
   ii. A taste enhancer, preferably saccharin sodium, at a quantity ranging from 0.05 to 3.00% w/w in the reconstitution liquid.
   iii. A preservative or system of preservatives known from the state of art, such as p-hydroxy benzoate esters or salts of sorbic acid, at quantities sufficient to prevent the growth of microbial load during the use of the reconstituted product.

The following examples illustrate the present invention without limiting its scope:

### EXAMPLES:

The examples presented herein fall in the following categories:
i. Examples of liquid formulations of levothyroxine showing that the stability of this particular active ingredient cannot be predicted based on the physicochemical characteristics of the materials used in the formulation, as well as that it is extremely unlikely to prepare aqueous liquid formulations of levothyroxine with an adequate shelf life to provide a commercially viable pharmaceutical form (Examples 1 a - 1 c).
ii. Examples that are actual embodiments of the present invention (Examples 2 and 3).

### Example 1:

Example 1 illustrates the problematic behaviour of levothyroxine in liquid formulations. Numerous solvents were tested with regard to their suitability for providing stable liquid formulations of levothyroxine, namely:
Example 1 a: Organic solvents and mixtures thereof with water. Testing was performed using:
   1. Glycerin 100%
   2. Propylene glycol 100%
   3. PEG 400 as is and in a 50% w/w mixture with water
   4. Solutol HS 15 (25%w/w)
   5. Cremophor EL (25%w/w)
   6. 1-methyl-Pyrrolidone (Pharmasolve) as is and in a 50%w/w mixture with water
   7. Soluphor P (2-pyrrolidone) as is and in a 50% w/w mixture with water
   8. Ethanol 100%
   9. Soluplus (10% w/w mixture with water)
Example 1b: Aqueous polymeric dispersions. Testing was performed using:
   1. Aqueous HPMC(E4M) 0,1 %, 0,5% and 1 %w/w
   2. Aqueous HPC 6cp pharmacoat 606 3%w/w and 6%w/w
   3. Aqueous HPMC 6cp E6LV 3%w/w and 6%w/w
   4. Aqueous PVP k30 5%w/w and 10%w/w
   5. Aqueous PEG 4000 (50%)
Example 1 c: Aqueous dispersions of sugars and similar substances. Testing was performed using:
   1. Sorbitol 85%
   2. Aqueous Sorbitol from liquid 85% w/v, 20%w/v and 40%w/v
   3. Aqueous Sorbitol from solid 20%w/v and 40%w/v
   4. Aqueous mannitol from solid 10%w/v
   5. Aqueous xylitol from solid 20%w/v and 40%w/v
   6. Aqueous maltodextrin from solid 20%w/v and 40%w/v

It was found that most solvents gave opaque solutions or solutions with visible precipitates, even though the concentration of the active ingredient was significantly lower than the saturation concentration estimated for the same solvents. In most cases a rapid and quantitative precipitation of the active ingredient, as well as degradation thereof into byproducts were observed, to an extent that did not allow the preparation of stable levothyroxine formulations.

None of the tested formulations of example 1 was found suitable for the preparation of a stable liquid dosage form of levothyroxine In addition, no pattern could be identified relating the physicochemical properties of the excipients used to the stability of the active ingredient, the confusion in this field as presented in the prior art thus being maintained.

Surprisingly, it was found that two carriers, sorbitol and maltodextrin optimized the stability of the active ingredient. However, these excipients alone are not capable of supporting a solid for reconstitution, as levothyroxine precipitates. The physical stability of the formulation during reconstitution was ensured by using aqueous solutions of polyvinyl pyrrolidone 5% w/w, which were found to solubilize the active ingredient for a period of time exceeding 3 months without affecting the stability of the formulation, in the presence of sorbitol or maltodextrin, thus making possible the preparation of an appropriate oral solution of the active ingredient after reconstitution. These unexpected results led to the concept of the system (kit) of the present invention.

Example 2: Example 2 is an embodiment of the present invention. The formulation is presented in table 1 (solid for reconstitution and reconstitution liquid). The composition of example 2 is presented in its reconstituted form, in which it is administered to the patient.

During the manufacturing process, the active ingredient and polyvinyl pyrrolidone are dispersed in water and are sprayed on solid sorbitol using a fluidized bed granulation process. After completion of the spraying step, the solid for reconstitution is dried under mild conditions in the same apparatus and is finally filled into amber glass vials.

The reconstitution liquid is manufactured via a very simple process, by dissolving polyvinyl pyrrolidone, the sweetener and the preservatives in water. The solution is filled into amber glass vials at an appropriate volume to allow the reconstitution of the solid.

| Table 1: Composition of a levothyroxine formulation based on the present invention. | | | | |
|---|---|---|---|---|
| Levothyroxine composition per vial of finished product | | | %w/v | Phase |
| Levothyroxine Sodium | 0,00375 | g | 0,00250 | Solid for reconstitution |
| Sorbitol | 14,69625 | g | 9,79750 | Solid for reconstitution |
| Polyvinyl pyrrolidone (as a binder) | 0,30000 | g | 0,20000 | Solid for reconstitution |
| Propyl parahydroxy benzoate sodium | 0,03000 | g | 0,02000 | Reconstitution liquid |
| Methyl parahydroxy benzoate sodium | 0,30000 | g | 0,20000 | Reconstitution liquid |
| Saccharin sodium | 1,50000 | g | 1,00000 | Reconstitution liquid |
| Polyvinyl pyrrolidone (as a solubilizer) | 7,50000 | g | 5,00000 | Reconstitution liquid |
| qs water | 150,00000 | ml | 100,00000 | Reconstitution liquid |

Prior to use, the reconstitution liquid is added to the solid for reconstitution and the oral solution is obtained by shaking.

Example 3: Example 3 is also an embodiment of the present invention. The formulation is presented in table 2 (solid for reconstitution and reconstitution liquid). The composition of example 3 is presented in its reconstituted form, in which it is administered to the patient.

| Table 2: Composition of a levothyroxine formulation based on the present invention. | | | | |
|---|---|---|---|---|
| Levothyroxine composition per vial of finished product | | | %w/v | Phase |
| Levothyroxine Sodium | 0,00375 | g | 0,00250 | Solid for reconstitution |
| Maltodextrin | 14,69625 | g | 9,79750 | Solid for reconstitution |
| Polyvinyl pyrrolidone (as a binder) | 0,30000 | g | 0,20000 | Solid for reconstitution |
| Propyl parahydroxy benzoate sodium | 0.03000 | g | 0,02000 | Reconstitution liquid |
| Methyl parahydroxy benzoate sodium | 0,30000 | g | 0,20000 | Reconstitution liquid |
| Saccharin sodium | 1,50000 | g | 1,00000 | Reconstitution liquid |
| Polyvinyl pyrrolidone (as a solubilizer) | 7,50000 | g | 5,00000 | Reconstitution liquid |
| qs water | 150,00000 | ml | 100,00000 | Reconstitution liquid |

During the manufacturing process, the active ingredient and Polyvinyl pyrrolidone are dispersed in water and are sprayed on solid maltodextrin using a fluidized bed granulation process. After completion of the spraying step, the solid for reconstitution is dried under mild conditions in the same apparatus and is finally filled into amber glass vials.

The reconstitution liquid is manufactured as described in example 2.

Prior to use, the reconstitution liquid is added to the solid for reconstitution and the oral solution is obtained by shaking.

## Claims

1. Stable pharmaceutical system (kit) for the preparation of oral solution of levothyroxine or pharmaceutically acceptable salt thereof, which consists of:
a) a solid for reconstitution in the form of powder or granules, which comprises levothyroxine or a pharmaceutically acceptable salt thereof as the active ingredient and at least one excipient-carrier of the active ingredient, preferably water soluble, and
b) a reconstitution liquid for the preparation of oral solution, which is aqueous and comprises at least one excipient for the solubilisation of the active ingredient.

2. Stable pharmaceutical system (kit) for the preparation of oral solution of levothyroxine or pharmaceutically acceptable salt thereof, according to claim 1, wherein levothyroxine content ranges from 0.01 to 1.00% w/w, preferably from 0.02 to 0.50% w/w and even more preferably from 0.02 to 0.1% w/w in the solid for reconstitution.

3. Stable pharmaceutical system (kit) for the preparation of oral solution of levothyroxine or pharmaceutically acceptable salt thereof, according to claims 1 or 2, wherein the excipient-carrier of the active ingredient is sorbitol or maltodextrin or a mixture thereof.

4. Stable pharmaceutical system (kit) for the preparation of oral solution of levothyroxine or pharmaceutically acceptable salt thereof, according to claim 3, wherein the amount of the excipient-carrier of the active ingredient ranges from 30.00 to 99.99% w/w and preferably from 97.90 to 99.48% w/w in the solid for reconstitution.

5. Stable pharmaceutical system (kit) for the preparation of oral solution of levothyroxine or pharmaceutically acceptable salt thereof, according to any of the preceding claims, wherein the solid for reconstitution further comprises other common excipients such as sweeteners, taste enhancers, water soluble lubricants, water soluble diluents, binders, viscosity regulators and pH adjusting systems.

6. Stable pharmaceutical system (kit) for the preparation of oral solution of levothyroxine or pharmaceutically acceptable salt thereof, according to any of the preceding claims, wherein the excipient for the solubilisation of the active ingredient in the aqueous reconstitution liquid is polyvinyl pyrrolidone.

7. Stable pharmaceutical system (kit) for the preparation of oral solution of levothyroxine or pharmaceutically acceptable salt thereof, according to claim 6, wherein the content of polyvinyl pyrrolidone ranges from 0.01 to 15.00% w/w and preferably from 2.50 to 7.50% w/w in the reconstitution liquid.

8. Stable pharmaceutical system (kit) for the preparation of oral solution of levothyroxine or pharmaceutically acceptable salt thereof, according to any of the preceding claims, wherein the reconstitution liquid further comprises other common excipients such as preservatives, sweeteners, taste enhancers, water soluble diluents, water soluble lubricants, viscosity regulators and pH adjusting systems.

9. Process for manufacturing the pharmaceutical system (kit) for the preparation of the oral solution of levothyroxine or pharmaceutically acceptable salt thereof, of claims 1-8, said process comprising:
a) the preparation of the solid for reconstitution by applying wet granulation and
b) the dissolution of excipients in water for the preparation of the reconstitution liquid.

10. Process for manufacturing the pharmaceutical system (kit) for the preparation of oral solution of levothyroxine or pharmaceutically acceptable salt thereof, according to claim 9, wherein:
a) during the wet granulation for the preparation of the solid for reconstitution, levothyroxine is dispersed in an aqueous solution of polyvinyl pyrrolidone and sprayed on maltodextrin or sorbitol or a mixture thereof in a fluidized bed, and the powders or granules resulting after spraying are dried in the same apparatus and
b) during the preparation of the reconstitution liquid, polyvinyl pyrrolidone, a taste enhancer, preferably a sweetener, and the preservatives, preferably p-hydroxy benzoate esters or salts of sorbic acid, are dissolved in water.

## Patentansprüche

1. Stabiles pharmazeutisches System (Kit) zur Bereitstellung einer oralen Lösung von Levothyroxin oder einem pharmazeutisch unbedenklichen Salz davon, bestehend aus:
a) einem Feststoff zur Rekonstitution in Form von Pulver oder Granulat, der Levothyroxin oder ein pharmazeutisch unbedenkliches Salz davon als Wirkstoff und mindestens einen Hilfsstoff-Träger des Wirkstoffes, vorzugsweise wasserlöslichen, umfasst und
b) einer Rekonstitutionsflüssigkeit zur Bereitstellung von oralen Lösung, die wässrig ist und mindestens einen Hilfsstoff zur Solubilisierung des Wirkstoffes umfasst.

2. Stabiles pharmazeutisches System (Kit) zur Bereitstellung einer oralen Lösung von Levothyroxin oder einem pharmazeutisch unbedenklichen Salz davon nach Anspruch 1, worin der Levothyroxingehalt im Feststoff zur Rekonstitution im Bereich von 0,01 bis 1,00% w/w, vorzugsweise von 0,02 bis 0,50% w/w und noch stärker bevorzugt von 0,02 bis 0, 1 % w/w liegt.

3. Stabiles pharmazeutisches System (Kit) zur Bereitstellung einer oralen Lösung von Levothyroxin oder einem pharmazeutisch unbedenklichen Salz davon nach den Ansprüchen 1 oder 2, worin der Hilfsstoff-Träger des Wirkstoffes Sorbit oder Maltodextrin oder eine Mischung davon ist.

4. Stabiles pharmazeutisches System (Kit) zur Bereitstellung einer oralen Lösung von Levothyroxin oder einem pharmazeutisch unbedenklichen Salz davon nach Anspruch 3, worin der Betrag des Hilfsstoffes-Trägers des Wirkstoffes im Feststoff zur Rekonstitution im Bereich von 30,00 bis 99,99% w/w und vorzugsweise von 97,90 bis 99,48% w/w liegt.

5. Stabiles pharmazeutisches System (Kit) zur Bereitstellung einer oralen Lösung von Levothyroxin oder einem pharmazeutisch unbedenklichen Salz davon, nach einem der vorhergehenden Ansprüche, worin der Feststoff zur Rekonstitution auch andere übliche Hilfsstoffe wie Süßstoffe, Geschmacksverstärker, wasserlösliche Gleitmittel, wasserlösliche Verdünnungsmittel, Bindemittel, Viskositätsregler und pH-Verstellsysteme umfasst.

6. Stabiles pharmazeutisches System (Kit) zur Bereitstellung einer oralen Lösung von Levothyroxin oder einem pharmazeutisch unbedenklichen Salz davon, nach einem der vorhergehenden Ansprüche, worin der Hilfsstoff zur Solubilisierung des Wirkstoffes in der wässrigen Rekonstitutionsflüssigkeit Polyvinylpyrrolidon ist.

7. Stabiles pharmazeutisches System (Kit) zur Bereitstellung einer oralen Lösung von Levothyroxin oder einem pharmazeutisch unbedenklichen Salz davon nach Anspruch 6, worin der Gehalt der Rekonstitutionsflüssigkeit an Polyvinylpyrrolidon im Bereich von 0,01 bis 15,00% w/w und vorzugsweise von 2,50 bis 7,50% w/w liegt.

8. Stabiles pharmazeutisches System (Kit) zur Bereitstellung einer oralen Lösung von Levothyroxin oder einem pharmazeutisch unbedenklichen Salz davon, nach einem der vorhergehenden Ansprüche, worin die Rekonstitutionsflüssigkeit auch andere übliche Hilfsstoffe, wie Konservierungsmittel, Süßstoffe, Geschmacksverstärker, wasserlösliche Verdünnungsmittel, wasserlösliche Gleitmittel, Viskositätsregler und pH-Verstellsysteme umfasst.

9. Verfahren zur Herstellung des pharmazeutischen Systems (Kits) zur Bereitstellung der oralen Lösung von Levothyroxin oder einem pharmazeutisch unbedenklichen Salz davon der Ansprüche 1-8, worin das gesagte Verfahren:
a) die Herstellung des Feststoffes zur Rekonstitution durch die Anwendung Feuchtgranulation und
b) die Auflösung der Hilfsstoffe in Wasser zur Bereitstellung der Rekonstitutionsflüssigkeit,
umfasst.

10. Verfahren zur Herstellung des pharmazeutischen Systems (Kits) zur Bereitstellung der oralen Lösung von Levothyroxin oder einem pharmazeutisch unbedenklichen Salz davon, nach Anspruch 9, worin:
a) während der Feuchtgranulation zur Herstellung des Feststoffes zur Rekonstitution Levothyroxin in einer wässrigen Lösung von Polyvinylpyrrolidon dispergiert und auf Maltodextrin oder Sorbitol oder einer Mischung davon in einer Wirbelschicht angespritzt wird, und die nach dem Sprühen erhaltenen Pulver oder Granulate in der gleichen Vorrichtung getrocknet werden und
b) während der Bereitstellung der Rekonstitutionsflüssigkeit, Polyvinylpyrrolidon, ein Geschmacksverstärker, vorzugsweise ein Süßstoff, und die Konservierungsmittel, vorzugsweise p-Hydroxybenzoatester oder Salze der Sorbinsäure, in Wasser gelöst werden.

## Revendications

1. Système pharmaceutique stable (kit) pour la préparation de solution orale de lévothyroxine ou d'un sel pharmaceutiquement acceptable de celle-ci, qui consiste en:
a) un solide de reconstitution sous forme de poudre ou de granules, qui comprend lévothyroxine ou un sel pharmaceutiquement acceptable de celle-ci comme principe actif et au moins un excipient-porteur du principe actif, de préférence soluble dans l'eau, et
b) un liquide de reconstitution pour la préparation de solution orale, qui est aqueux et comprend au moins un excipient pour la solubilisation du principe actif.

2. Système pharmaceutique stable (kit) pour la préparation de solution orale de lévothyroxine ou d'un sel pharmaceutiquement acceptable de celle-ci, selon la revendication 1, où la teneur en lévothyroxine du solide de reconstitution est comprise entre 0,01 et 1,00% w/w, de préférence entre 0,02 et 0,50% w/w et encore plus préférentiellement entre 0,02 et 0,1% w/w.

3. Système pharmaceutique stable (kit) pour la préparation de solution orale de lévothyroxine ou d'un sel pharmaceutiquement acceptable de celle-ci, selon les revendications 1 ou 2, dans lequel l'excipient-porteur du principe actif est le sorbitol ou la maltodextrine ou un mélange de ceux-ci.

4. Système pharmaceutique stable (kit) pour la préparation de solution orale de lévothyroxine ou d'un sel pharmaceutiquement acceptable de celle-ci, selon la revendication 3, où la quantité de l'excipient-porteur du principe actif dans le solide de reconstitution est comprise entre 30,00 et 99,99% w/w et de préférence entre 97,90 et 99,48% w/w.

5. Système pharmaceutique stable (kit) pour la préparation de solution orale de lévothyroxine ou d'un sel pharmaceutiquement acceptable de celle-ci, selon l'une quelconque des revendications précédentes, dans lequel le solide de reconstitution comprend en outre d'autres excipients communs tels que des édulcorants, des exhausteurs de goût, des lubrifiants solubles dans l'eau, des diluants solubles dans l'eau, des liants, des régulateurs de viscosité et des systèmes de réglage du pH.

6. Système pharmaceutique stable (kit) pour la préparation de solution orale de lévothyroxine ou d'un sel pharmaceutiquement acceptable de celle-ci, selon l'une quelconque des revendications précédentes, où l'excipient pour la solubilisation du principe actif dans le liquide aqueux de reconstitution est la polyvinylpyrrolidone.

7. Système pharmaceutique stable (kit) pour la préparation de solution orale de lévothyroxine ou d'un sel pharmaceutiquement acceptable de celle-ci, selon la revendication 6, où la teneur en polyvinylpyrrolidone du liquide de reconstitution est comprise entre 0,01 et 15,00% w/w et de préférence entre 2,50 et 7,50% w/w.

8. Système pharmaceutique stable (kit) pour la préparation de solution orale de lévothyroxine ou d'un sel pharmaceutiquement acceptable de celle-ci, selon l'une quelconque des revendications précédentes, dans lequel le liquide de reconstitution comprend en outre d'autres excipients communs tels que des conservateurs, des édulcorants, des exhausteurs de goût, des diluants solubles dans l'eau, des lubrifiants solubles dans l'eau, des régulateurs de viscosité et des systèmes de réglage du pH.

9. Procédé de fabrication du système pharmaceutique (kit) pour la préparation de solution orale de lévothyroxine ou d'un sel pharmaceutiquement acceptable de celle-ci des revendications 1-8, ledit procédé comprenant:
a) la préparation du solide de reconstitution en appliquant la granulation par voie humide et
b) la dissolution dans l'eau des excipients pour la préparation du liquide de reconstitution.

10. Procédé de fabrication du système pharmaceutique (kit) pour la préparation de solution orale de lévothyroxine ou d'un sel pharmaceutiquement acceptable de celle-ci, selon la revendication 9, dans lequel:
a) au cours de la granulation par voie humide pour la préparation du solide de reconstitution, la lévothyroxine est dispersée dans une solution aqueuse de polyvinylpyrrolidone et pulvérisée sur de la maltodextrine ou du sorbitol ou un mélange de ceux-ci dans un lit fluidisé, et les poudres ou les granulés résultant de la pulvérisation sont séchés dans le même appareil et
b) au cours de la préparation du liquide de reconstitution, la polyvinylpyrrolidone, un exhausteur de goût, de préférence un édulcorant, et les agents conservateurs, de préférence esters de p-hydroxybenzoate ou sels de l'acide sorbique, sont dissous dans l'eau.
